# EUROPEAN PATENT SPECIFICATION

(11) **EP 4 547 118 B1**
(45) Date of publication and mention of the grant of the patent: **21.10.2026**
(21) Application number: 22748520.8
(22) Date of filing: 28.06.2022
(51) Int. Cl.: A61B 10/02, A61B 17/34, A61B 90/00

(54) **BIOPSY DEVICES HAVING DEPLOYABLE MARKERS**
BIOPSIEVORRICHTUNGEN MIT EINSETZBAREN MARKERN
DISPOSITIFS DE BIOPSIE AYANT DES MARQUEURS DÉPLOYABLES

(43) Date of publication of application: 07.05.2025
(73) Proprietor: Bard Peripheral Vascular, Inc., Franklin Lakes, NJ 07417 (US)
(72) Inventor: GLASPIE, Koltin, Franklin Lakes, New Jersey 07417 (US); CHANDUSZKO, Andrzej, Franklin Lakes, New Jersey 07417 (US)
(74) Representative: Hoffmann Eitle
(86) International application number: PCT/US2022/035240
(87) International publication number: WO 2024/005783

(56) References cited:
- US-A1- 2010 286 627
- US-A1- 2011 184 280
- US-A1- 2018 168 770
- US-A1- 2020 187 921
- US-B1- 6 350 244
- US-B2- 8 167 817
- US-B2- 8 721 563

## Description

### TECHNICAL FIELD

The present specification generally relates to biopsy devices and, more specifically, to biopsy devices having deployable markers for marking a location of a tissue biopsy.

### BACKGROUND

In diagnosing and treating certain medical conditions, it is often desirable to perform a biopsy, in which a specimen or sample of tissue is removed for pathological examination, tests, and/or analysis. During biopsy procedures and lumpectomy procedures, tissue markers may be implanted to mark the location of the biopsy and/or the lesion or diseased tissue targeted for biopsy. These tissue markers may be later used during follow-up procedures or appointments to locate the original biopsy location. The markers may be detectable by a scan or other procedure on the subject to view a target site where tissue was removed. The markers may be deployed via a marking device. However, this requires a separate device from the biopsy device, increasing the number of parts and time required to remove tissue and mark the location.

US 2018/168770 A1 discloses a device for marking the location of a tissue biopsy. The device includes a needle with a proximal end, a distal end, a slot disposed adjacent the distal end, and a tip having an opening. The device further includes a handle coupled to the proximal end of the needle, and a cutting apparatus. The cutting apparatus includes a cutting mechanism disposed within the needle and adapted to remove tissue from a target area. The device further includes an ejector mechanism having an ejector rod disposed within the needle and an actuator disposed on the handle and operatively coupled to the ejector rod. The actuator is actuated to eject a marker disposed within the distal end of the needle through one or more of the slot or the opening to mark the location of the tissue biopsy.

US 2010/286627 A1 discloses a biopsy marker deployment device adapted to selectively deposit a marker in a target location, such as a biopsy site. The device includes a deployment assembly comprising a cannula adapted to house at least one marker, an outlet aperture defined by a portion of the cannula, and an actuatable pushrod slidably disposed and movable within the cannula. The deployment assembly further comprises a selectively opening outlet door movable between an open position and a closed position. The outlet door is biased in the closed position and at least partially obstructs the aperture in the closed position to prevent a marker from reentering the cannula upon deployment.

US 8 721 563 B2 discloses a single-insertion, multiple sampling device with integrated marker release.

### SUMMARY

In one embodiment, a biopsy device for marking a location of a tissue biopsy, the biopsy device including: a needle having a needle body and a needle tip movably coupled to the needle body, a cutting apparatus disposed within the needle body, and a marker. The needle tip defines an open lumen. The marker is positioned within the open lumen. The marker includes a marker pellet. The needle tip is movable between a undeployed position and a deployed position in which the needle tip is positioned closer to the cutting apparatus than in the undeployed position such that the cutting apparatus interacts with the marker to push the marker pellet through the open lumen.

In an illustrative non-claimed embodiment, a biopsy device for marking a location of a tissue biopsy, the biopsy device includes: a needle having a needle body and a needle tip coupled to the needle body, one or more markers, and a push rod disposed within and movable along the needle body. The needle body defines a central lumen therethrough, and the needle tip defines one or more open lumens extending therethrough to the central lumen. The central lumen defines a center axis. The one or more markers are positioned within the one or more open lumens, each marker including a marker pellet. The push rod is movable between a retracted position and a deployment position. In the retracted position, the push rod is spaced apart from the one or more markers. In the deployment position, the push rod interacts with the one or more markers to push the marker pellet of the one or more markers through the one or more open lumens to deliver the marker pellet to a target site.

In an illustrative non-claimed method of using a biopsy device, the method including: positioning the biopsy device at a target site, moving a cutting apparatus into a closed position to remove a tissue sample from the target site; and moving a needle tip, while the cutting apparatus is positioned in the closed position, to a deployed position, thereby causing the cutting apparatus to interact with a marker to push a marker pellet through an open lumen to deliver the marker pellet to the target site. The biopsy device includes: a needle having a needle body and the needle tip movably coupled to the needle body, the needle tip defining the open lumen therethrough, the cutting apparatus disposed within the needle body, and the marker positioned within the open lumen, the marker including the marker pellet.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1 schematically depicts a perspective view of an illustrative biopsy device according to one or more embodiments shown and described herein;
FIG. 2 schematically depicts a perspective view of a distal end of an illustrative needle of the biopsy device of FIG. 1, according to one or more embodiments shown and described herein;
FIG. 3A schematically depicts a cross sectional side view of the distal end of the needle of FIG. 2 taken along lines A-A with the biopsy device in an undeployed state, according to one or more embodiments shown and described herein;
FIG. 3B schematically depicts a cross sectional side view of the distal end of the needle of FIG. 2 taken along lines A-A with the biopsy device in a deployed state, according to one or more embodiments shown and described herein;
FIG. 4A schematically depicts a cross sectional side view of another illustative non-claimed needle of the biopsy device of FIG. 1 with the biopsy device in an undeployed state;
FIG. 4B schematically depicts a cross sectional side view of the needle of FIG. 4A with the biopsy device in a deployed state;
FIG. 5A schematically depicts a cross sectional side view of yet another illustrative non-claimed needle of the biopsy device of FIG. 1 with the biopsy device in an undeployed state;
FIG. 5B schematically depicts a cross sectional side view of the needle of FIG. 5A with the biopsy device in a deployed state;
FIG. 6 depicts a block diagram of an illustrative control system for controlling operation of a biopsy device; and
FIG. 7 depicts a flowchart of an illustrative non-claimed method of operating a biopsy device.

### DETAILED DESCRIPTION

The present disclosure, in one form, is related to a biopsy device for removing a tissue sample from a subject and marking the location of the removed tissue. The biopsy devices described herein generally include a needle, a cutting apparatus, and a plurality of markers. The biopsy devices described herein are configured such that a tissue sample may be taken from a subject and a marker may be deployed to mark the location of where the tissue was removed. The biopsy device incorporates components for both removing a tissue sample and marking the location. As such, the procedure may be performed without inserting a separate marking device to mark the location. To achieve this, the needle includes a needle body, a needle tip that defines an opening into a recess of the needle body that releasably holds a marker therein, and cutting apparatus for removing the tissue sample from the subject. The cutting apparatus is slidably disposed within the needle body such that the cutting apparatus is slidably movable along a length of the body of the needle to cut the tissue sample from a subject that has been received within an inner lumen of the needle. The needle tip is movably coupled to the needle body to allow the needle tip to move relative to the needle body. The needle tip may move toward the needle body such that a portion of the cutting apparatus contacts the marker after cutting the tissue sample to push the marker out of the opening of the needle tip, thereby marking a location of the target site. Various embodiments of the biopsy device and the operation of the biopsy device will be described in more detail herein.

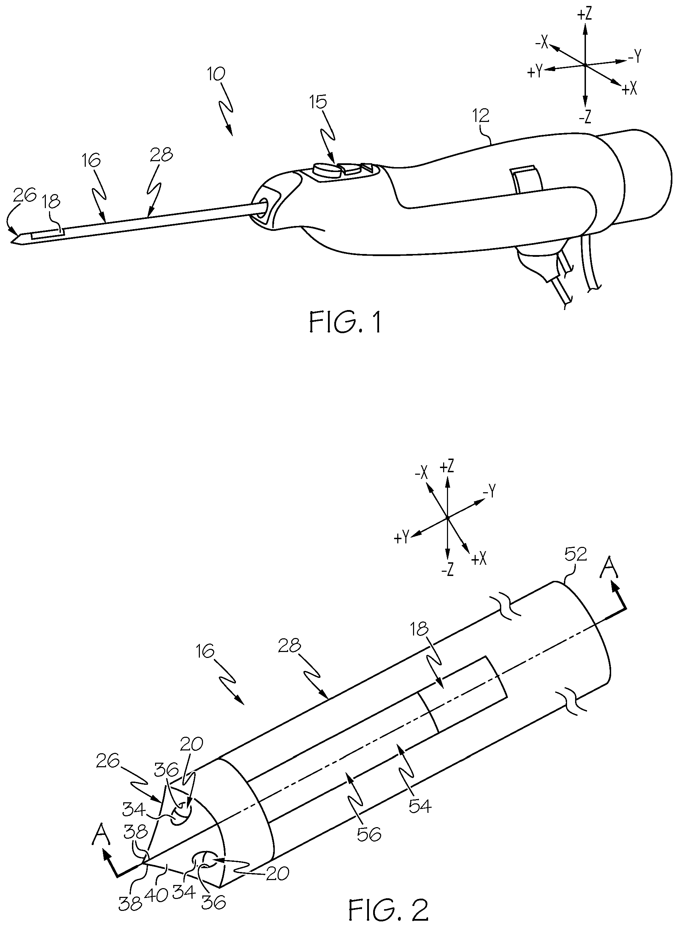

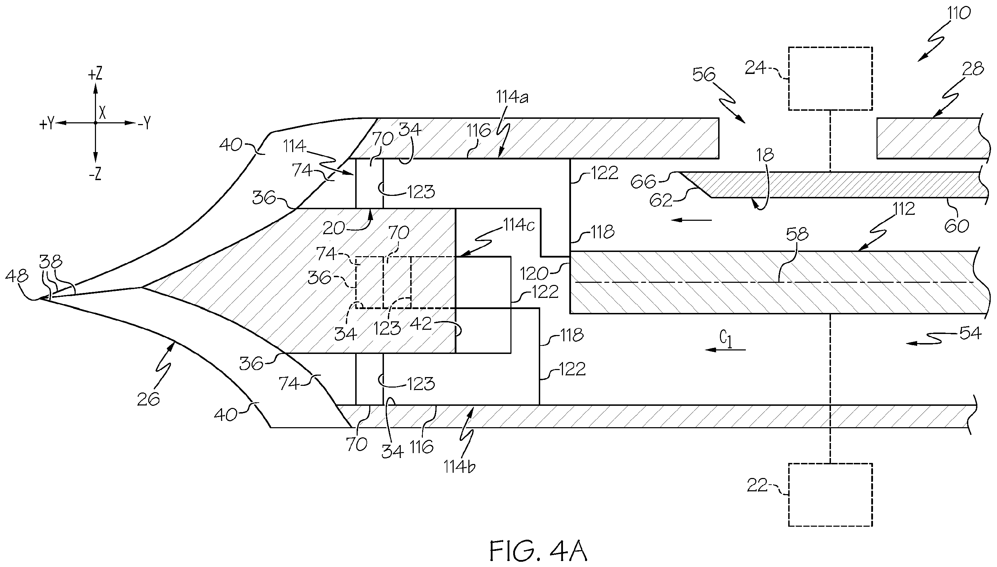

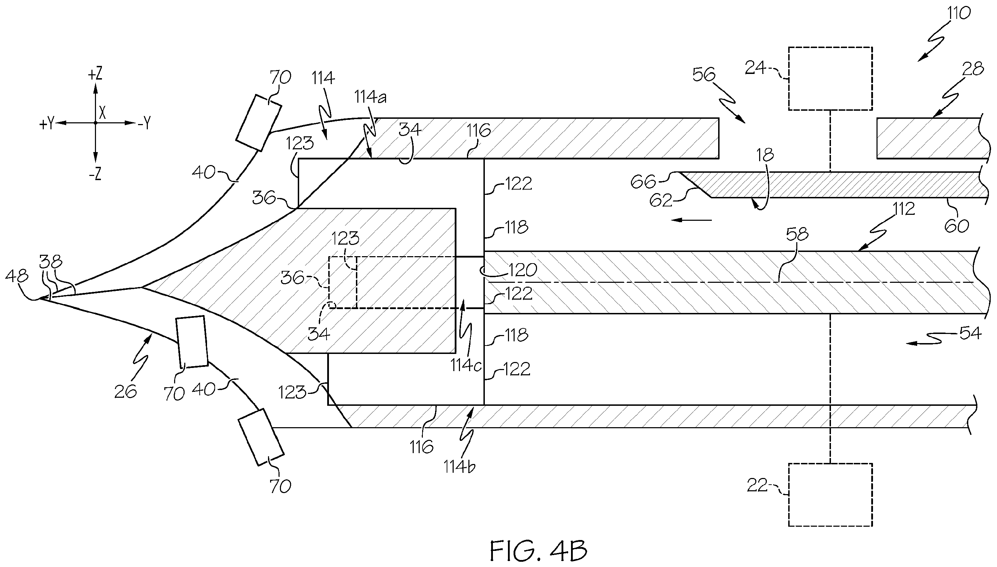

Referring now to FIGS. 1, 2, and 3A, an illustrative biopsy device 10 for removing a tissue sample and marking the location of the removed tissue sample of a subject is depicted. The biopsy device 10 may include a handle 12, a needle 16, a cutting apparatus 18, and one or more markers 20. Referring to FIGS. 1 and 4, the handle 12 may house a control system 14 for operating the biopsy device 10. As will be described in further detail herein, the control system 14 may include a first actuator 22 and a second actuator 24 that are configured to operate components of the needle 16 and the cutting apparatus 18 to remove the tissue sample and deploy one of the markers 20. The control system 14 may further include a plurality of inputs 15 positioned on the handle 12 that are configured to initiate and cease operation of the first actuator 22 and the second actuator 24.

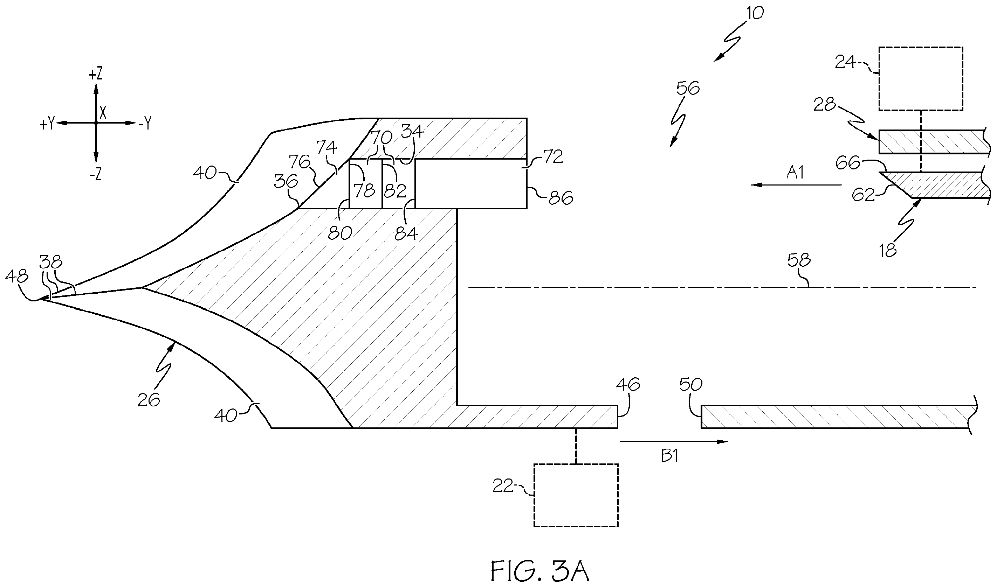

Referring to FIGS. 2 and 3A, the needle 16 includes a needle tip 26 and a needle body 28. The needle tip 26 may be formed separate from the needle body 28 such that the needle tip 26 is movable relative to the needle body 28. The needle body 28 may include a proximal end 52 and an opposite distal end 50. The proximal end 52 of the needle body 28 may be fixedly coupled to the handle 12, and the needle body 28 may extend away from the handle 12 in a longitudinal direction (e.g., in the +Y direction) such that the distal end 50 of the needle body 28 is spaced apart from the handle 12. The needle body 28 may define a central lumen 54 that extends from the proximal end 52 to the distal end 50, and an opening 56 formed therein. The central lumen 54 may define a center axis 58 extending along a length of the needle body 28 through a center of the needle body 28. The opening 56 may extend transverse to the center axis 58 through the needle body 28 and into the central lumen 54. The opening 56 may extend a first distance from the proximal end 52 of the needle body 28 toward the distal end 50. The first distance may be any distance suitable to receive an amount of tissue necessary for a biopsy. The needle body 28 may include any cross sectional shape capable of enclosing an area therein, such as a cylinder, a rectangle, or the like.

The needle tip 26 may be positioned at the distal end 50 of the needle body 28 such that the needle body 28 is disposed between the needle tip 26 and the handle 12. The needle tip 26 may define a plurality of open lumens 34 extending therethrough and a plurality of openings 36. The needle tip 26 may include a plurality of edges 38, a plurality of surfaces 40 that adjoin along the plurality of edges 38, a distal tip 48, and a proximal end 46 opposite the distal tip 48. The plurality of edges 38 may be sharpened such that the needle tip 26 is configured to puncture tissue and create an access path (e.g., a proposed biopsy tract) in the tissue at least to the target site. The openings 36 of the needle tip 26 may be formed in the plurality of surfaces 40 at an interface between the open lumens 34 and the surfaces 40. The open lumens 34 may be open to an environment external to the biopsy device 10, such as a target site in a patient's body. The open lumens 34 may be open at the openings 36 to allow an object to exit the needle tip 26 by passing through the open lumen 34 and out through the opening 36.

The plurality of open lumens 34 may extend through the needle tip 26 to the central lumen 54 defined by the needle body 28 such that an object in the central lumen 54 may interact with an object positioned in the open lumens 34 defined by the needle tip 26. The needle tip 26 may include a number of openings 36 equal to a number of open lumens 34 with the openings 36 arranged such that each opening 36 interfaces with one of the open lumens 34. The openings 36 may be radially spaced apart from the center axis 58 such that the openings 36 are radially offset from the center axis 58 in the lateral direction (e.g., in the +/- X direction) and the vertical direction (e.g., in the +/- Z direction). The needle tip 26 may include any operable number of openings 36 and lumens 34 for deploying markers therethrough, such as, for example, one, two, three, or more than three. The plurality of openings 36 may be positioned adjacent the distal tip 48 of the needle tip 26 with the openings 36 arranged such that one of the edges 38 is positioned between each adjacent pair of openings 36.

The distal end 50 of the needle body 28 may be movably coupled to the proximal end 46 of the needle tip 26 such that the needle tip 26 may move relative to the needle body 28 in the longitudinal direction (e.g., in the +/- Y direction). The needle tip 26 may be movably coupled to the needle body 28 via the first actuator 22 such that actuation of the first actuator 22 moves the needle tip 26 toward or away from the needle body 28. The first actuator 22 may be operatively coupled to the needle tip 26 to move the needle tip 26 relative to the needle body 28. For example, the first actuator 22 may be a linear actuator that is fixedly coupled between the needle tip 26 and the needle body 28 such that actuation of the first actuator 22 extends and retracts the needle tip 26 relative to the needle body 28. However, the first actuator 22 may be any actuator capable of moving the needle tip 26 linearly, such as a linear actuator, a hydraulic actuator, a pneumatic actuator, or the like. Other embodiments for movably coupling the needle tip 26 to the needle body 28 are contemplated and possible such as, for further example, the needle tip 26 may alternatively be movably coupled to the needle body 28 via another mechanism, such as a pin and slot. In a pin and slot mechanism, a pin extends from one of the needle body 28 and the needle tip 26 to extend into a slot formed in the other of the needle body 28 and the needle tip 26.

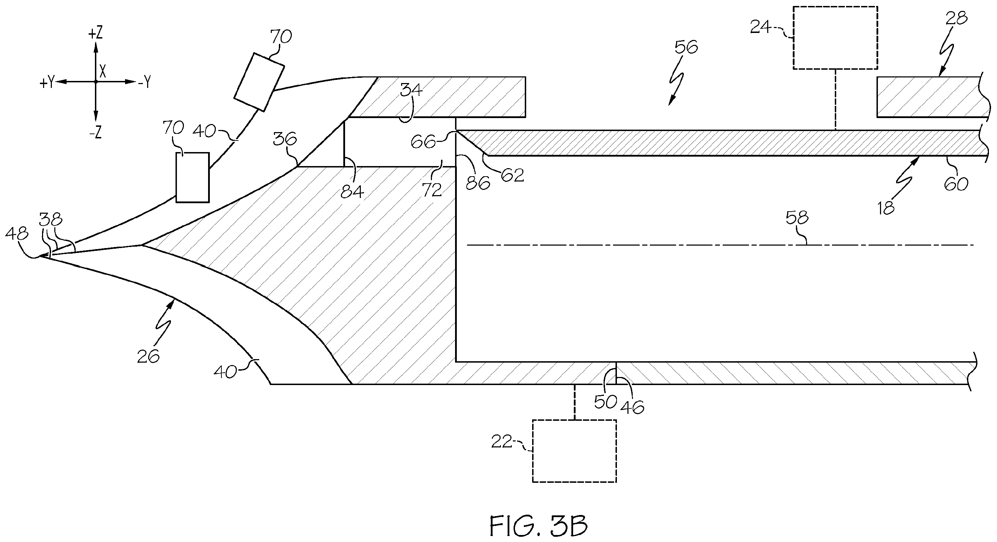

Referring to FIGS. 3A and 3B, each of the one or more markers 20 may include a marker pellet 70, a push pellet 72, and a resorbable pellet 74. In embodiments, the markers 20 may include a plurality of marker pellets 70. Each of the marker pellet 70, the push pellet 72, and the resorbable pellet 74 may be positioned within the respective lumen 34 of the plurality of open lumens 34 with the push pellet 72 positioned proximal to (e.g., in the -Y direction) the marker pellet 70 and the resorbable pellet 74 positioned distal to (e.g., in the +Y direction) the marker pellet 70. In embodiments having a plurality of markers 20, each marker 20 may be positioned in a respective one of the plurality of open lumens 34 such that one marker 20 is positioned in each lumen 34. The push pellet 72 may have a length greater than a length of the marker pellet 70 and/or a length of the resorbable pellet 74. The length of the push pellet 72 may be large enough such that the push pellet 72 extends proximally (e.g., in the -Y direction) from the open lumen 34 toward the cutting apparatus 18 and into the central lumen 54 of the needle body 28 allowing the cutting apparatus 18 to push the marker pellets 70 out of the open lumen 34 through the opening 36. The length of the push pellet 72 may be substantially the same as a length of the open lumen 34 so that when the push pellet 72 is positioned entirely within the open lumen 34, the marker pellets 70 positioned distal to the push pellet 72 are deployed out of the opening 36.

The resorbable pellet 74 may be positioned distal to the marker pellet 70 within the open lumen 34 between the opening 36 in the needle tip 26 and the marker pellet 70. The resorbable pellet 74 may be coupled to the needle tip 26 within the open lumen 34 to seal the opening 36 for a predetermined period of time prior to resorption of the resorbable pellet 74, thereby restricting the marker pellets 70 from exiting the open lumen 34 through the opening 36. The resorbable pellet 74 may be coupled to the needle tip 26 by, for example, a press fit. The resorbable pellet 74 may be configured to be resorbed by a body of the subject when the biopsy device 10 is positioned within the body. The resorbable pellet 74 may be formed of a bio-resorbable material, where "bio-resorbable" as used herein means resorbable in a biological environment, such as within the body of a patient. A bio-resorbable material is thus a material which may be absorbed, dissolved, broken down, degraded, assimilated, or otherwise biologically removed from a biological environment, such as from within the body of a patient, via interaction with the biological environment. Once the resorbable pellet 74 is resorbed, the marker pellet 70 may move along the open lumen 34 and through the opening 36 unrestricted by the resorbable pellet 74.

The marker pellet 70 may include a first end 80 and an opposite second end 82. The marker pellet 70 may be formed of a material that is detectable from outside of the body of a subject, such as, for example, materials that are visible via imaging (e.g., a radiopaque material or the like). Specific shapes of the marker pellet 70 may include, for example, a coil, heart, ribbon, hourglass, or any other operable shape capable of being detected during an imaging procedure. Specific materials of the marker pellet 70 may include, for example, titanium, stainless steel, carbon-coated ceramic, carbon-coated zirconium oxide, nitinol, poly(L-lactide-co-ε-caprolactone), polylactic acid (*PLA*), polycaprolactone (PCL), a bioresorbable material containing radiopaque additives such as iodine or barium sulfate, or any other operable material that is biocompatible with the subject. The first end 80 of the marker pellet 70 may be positioned adjacent to and contact the second surface 78 of the resorbable pellet 74. In embodiments, the marker 20 may include a plurality of marker pellets 70 each positioned between the push pellet 72 and the opening 36. In embodiments having a plurality of marker pellets 70, the marker pellets 70 may be arranged such that the second end 82 of one of the plurality of marker pellets 70 contacts or is positioned adjacent to the first end 80 of an adjacent marker pellet 70.

The push pellet 72 may include a first end 84 and an opposite second end 86. The push pellet 72 may be at least partially positioned within the open lumen 34 in the piercing structure 32 of the needle tip 26. The push pellet 72 may extend from the open lumen 34 past the interior surface 42 of the piercing structure 32 of the needle tip 26 into the central lumen 44. The first end 84 of the push pellet 72 may be positioned adjacent to and contact the second end 82 of the marker pellet 70. The push pellet 72 may be shaped such that the push pellet 72 may move the marker pellet 70 along the open lumen 34 when the push pellet 72 is moved. Each of the resorbable pellet 74, the marker pellet 70, and the push pellet 72 may be press fit or otherwise coupled to the central lumen 54 of the needle tip 26 such that the resorbable pellet 74, the marker pellet 70, and the push pellet 72 are maintained within the open lumen 34.

The cutting apparatus 18 may a distal end 62 and an opposite proximal end (not shown). The cutting apparatus 18 may include a shape similar to that of the needle body 28, such as a cylindrical shape. The distal end 62 may be chamfered or beveled such that the cutting apparatus 18 includes an edge 66 at the distal end 62. The edge 66 may be configured to puncture and separate tissue from the subject to position the tissue within the biopsy device 10 for removal from the subject. The second end 86 of the push pellet 72 may be angled to complement the shape of the distal end 62 of the cutting apparatus 18 such that, when the cutting apparatus 18 contacts the push pellet 72, the second end 86 of the push pellet 72 may be coplanar with the distal end 62 of the cutting apparatus 18. The flush contact between the push pellet 72 and the cutting apparatus 18 may reduce, minimize, or eliminate the wear on the edge 66 of the cutting apparatus 18 caused by repeated contact between the cutting apparatus 18 and the push pellet 72.

The cutting apparatus 18 may be disposed within the central lumen 54 in the needle body 28. The cutting apparatus 18 may be movable within the needle body 28 between an open position (FIG. 3A) and a closed position (FIG. 3B). The second actuator 24 may be operatively coupled to the cutting apparatus 18 to move the cutting apparatus 18 between the open position and the closed position. The second actuator 24 may be any actuator capable of moving the cutting apparatus 18 linearly, such as a linear actuator, a hydraulic actuator, a pneumatic actuator, or the like. The cutting apparatus 18 may travel a second distance when moving from the open position to the closed position. The second distance may be greater than the first distance that the opening 56 extends such that the cutting apparatus 18 may extend across the opening 56 when moving from the open position to the closed position. In embodiments, the cutting apparatus 18 may be capable of extending into the open lumen 34 in the needle tip 26 such that the cutting apparatus 18 contacts one of the push pellet 72 and the marker pellet 70. In such embodiments, the biopsy device 10 may not include a push pellet 72 such that the cutting apparatus 18 may directly contact one of the marker pellets 70.

Referring to FIG. 3A, in the open position, the cutting apparatus 18 may be spaced apart from the opening 56 in the needle body 28. When moving from the open position toward the closed position, the cutting apparatus 18 may move toward the needle tip 26 in the direction of arrow A1 in the longitudinal direction (e.g., in the +Y direction). Referring to FIG. 3B, in the closed position, the cutting apparatus 18 may be positioned between and separate the opening 56 and the central lumen 54 in the needle body 28, thereby enclosing the central lumen 54 and cutting off access to the central lumen 54 through the opening 56. In the closed position, the edge 66 of the cutting apparatus 18 may be positioned between the distal end 50 of the needle body 28 and the needle tip 26. In embodiments, in the closed position, the edge 66 of the cutting apparatus 18 may extend into the open lumen 34 in the needle tip 26. Referring again to FIGS. 3A and 3B, the needle tip 26 may be movable relative to the needle body 28 between an undeployed position (FIG. 3A), and a deployed position (FIG. 3B) in which the needle tip 26 is positioned closer to the cutting apparatus 18 than in the undeployed position. In the undeployed position, the cutting apparatus 18 is spaced apart from the push pellet 72 with the marker pellets 70 positioned within the open lumen 34 defined by the needle tip 26. In the undeployed position, the proximal end 46 of the needle tip 26 may be spaced apart from the needle body 28 so that the needle tip 26 is movable in the direction of arrow B1. When moving from the undeployed position to the deployed position, the needle tip 26 moves in the direction of arrow B1. Arrow B1 extends in the longitudinal direction (e.g., in the -Y direction). In the deployed position, the marker pellets 70 are deployed to be positioned outside of the open lumen 34 defined by the needle tip 26 and positioned within a body of the subject. In the deployed position, the cutting apparatus 18 may interact with the push pellet 72 of the marker 20 to push the marker pellet 70 through the open lumen 34 and the opening 36 to deliver the marker pellet 70 to a target site. In the deployed position, the cutting apparatus 18 may contact the push pellet 72 to advance the push pellet 72 and the marker pellet 70 toward the opening 36 of the open lumen 34. In embodiments, the needle tip 26 may be biased toward the undeployed position to prevent the needle tip 26 from moving from the undeployed position to the deployed position as the biopsy device 10 is inserted into the subject's body. The needle tip 26 may be biased by any known biasing mechanism, such as, for example, a spring, a magnet, or the like.

Referring now to FIGS. 4A and 4B, an alternative non-claimed biopsy device 110 is depicted.
The alternative biopsy device 110 may include the needle 16, the cutting apparatus 18, and the marker 20 similar to the biopsy device 10 described above, and will not be described again for brevity. The needle tip 26 of the alternative biopsy device 110 may be fixedly coupled to the needle body 28 such that the needle tip 26 may not be movable relative to the needle body 28. The needle tip 26 and the needle body 28 may be formed together as a one-piece monolithic structure. In embodiments, the needle tip 26 and the needle body 28 may be formed separately and fixedly coupled together by, for example, welding, adhesive, epoxy, fasteners, or the like.

The alternative biopsy device 110 may include a push rod 112 and a plurality of alternative push pellets 114. The alternative push pellets 114 may each include a core 116 and a flange 118 extending from the core 116. The core 116 may be elongated along the longitudinal direction (e.g., in the +/- Y direction). The flange 118 may extend from the core 116 toward the center axis 58 defined by the central lumen 54 in the needle body 28. Accordingly, depending on the location of the respective lumen 34 that the push pellet 114 is positioned within, the flange 118 may extend from the core 116 in the vertical direction (e.g., in the +/-Z direction), the lateral direction (e.g., in the +/-X direction), or a combination thereof. The flange 118 may extend toward the center axis 58 such that an orthographic projection of the push rod 112 extending along the center axis 58 intersects the flange 118 of the push pellet 114.

The flange 118 may include a contact surface 122 disposed to face the central lumen 54 in the needle body 28, and the core 116 may include a pushing surface 123 opposite the contact surface 122 of the flange 118. The core 116 of the alternative push pellet 114 may be at least partially positioned within the open lumen 34 of the needle tip 26. The flange 118 may extend from the core 116 of the push pellet 114 to be positioned within the central lumen 54 of the needle body 28. The flange 118 may be spaced apart from the needle tip 26 such that the core 116 of the push pellet 114 may traverse the open lumen 34 in the needle tip 26 a predetermined distance before the flange 118 contacts the needle tip 26.

The plurality of push pellets 114 may each include a different length. Specifically, the biopsy device 110 may include a first push pellet 114a, a second push pellet 114b, and a third push pellet 114c, each being disposed in a different lumen 34. The first push pellet 114a may have a first length, the second push pellet 114b may have a second length, and the third push pellet 114c may have a third length. Each of the first length, the second length, and the third length may be defined by a distance between the pushing surface 123 and the contact surface 122 of the respective push pellet 114. The first length may be greater than the second length, and the second length may be greater than the third length such that the contact surface 122 of each of the push pellets 114 is offset in the longitudinal direction. The plurality of push pellets 114 may each be disposed in the plurality of open lumens 34 such that the pushing surface 123 of each of the respective cores 116 are disposed a same distance from the respective openings 36. Accordingly, the flange 118 of the push pellet 114a extends further into the central lumen 54 of the needle body 28 in the longitudinal direction (e.g., in the -Y direction) than the flange 118 of the push pellet 114b or the flange 118 of the push pellet 114c. Similarly, the flange 118 of the push pellet 114b extends further into the central lumen 54 in the longitudinal direction (e.g., in the -Y direction) than the flange 118 of the push pellet 114c. With the flanges 118 of the plurality of push pellets 114 being disposed at various distances from the needle tip 26 into the central lumen 54 of the needle body 28, the push rod 112 may contact the push pellets 114 at different distances to deploy the respective marker pellets 70 discretely.

The push rod 112 may be disposed within and movable along the needle body 28 in the longitudinal direction (e.g., in the +/- Y direction). The push rod 112 may include a distal end 120 and an opposite proximal end (not shown). The push rod 112 may be an elongated member such that the push rod 112 extends along the length of the needle body 28. The push rod 112 may extend from the handle 12 such that the proximal end of the push rod 112 is positioned within or adjacent to the handle 12. The distal end 120 of the push rod 112 may be configured to contact the push pellets 114 to eject the push pellets 114 from the opening 36 in the needle tip 26. The proximal end of the push rod 112 may be operatively coupled to the first actuator 22 such that the first actuator 22 may move the push rod 112 in the direction of arrow C1 between a retracted position and a deployment position.

Referring to FIG. 4A, in the retracted position, the distal end 120 of the push rod 112 may be spaced apart from the flange 118 of each of the push pellets 114. The push rod 112 may move toward the needle tip 26 in the longitudinal direction (e.g., in the +Y direction) in the direction of arrow C1 when moving from the retracted position toward the deployment position. Referring to FIG. 4B, in the deployment position, the distal end 120 of the push rod 112 may contact the contact surface 122 of the flange 118 of each of the push pellets 114 to move the push pellets 114 through the open lumen 34 of the needle tip 26 toward the opening 36. When moving between the retracted position and the deployment position, the push rod 112 contacts the flange 118 of the push pellet 114a before contacting the flange 118 of either of the push pellet 114b or the push pellet 114c. Further movement of the push rod 112 toward the deployment position moves the push pellet 114a in the longitudinal direction (e.g., in the +Y direction) to deploy the respective marker pellet 70. Once the marker pellet 70 in contact with the push pellet 114a is deployed, the push rod 112 may contact the flange 118 of the push pellet 114b. The push rod 112 may similarly be continually advanced toward the deployment position to move the push pellet 114b in the longitudinal direction (e.g., in the +Y direction) to deploy the respective marker pellet 70. Once the marker pellet 70 in contact with the push pellet 114b is deployed, the push rod 112 may contact the flange 118 of the push pellet 114c. The push rod 112 may then be advanced to the deployment position to move the push pellet 114c in the longitudinal direction (e.g., in the +Y direction) to deploy the respective marker pellet 70. Accordingly, the alternative biopsy device 110 may deploy the marker pellets 70 at different times and locations, such as when taking multiple tissue samples from different target sites.

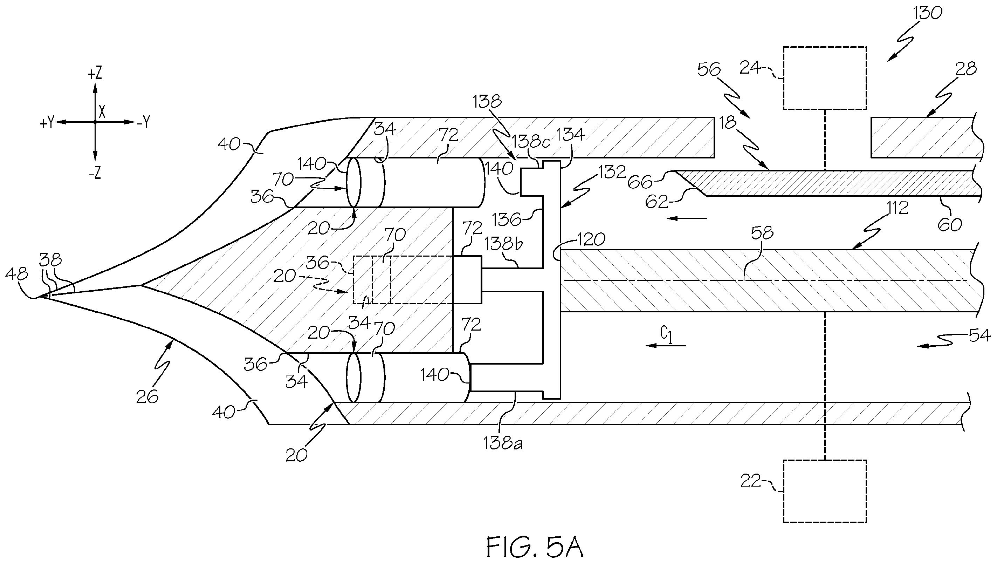

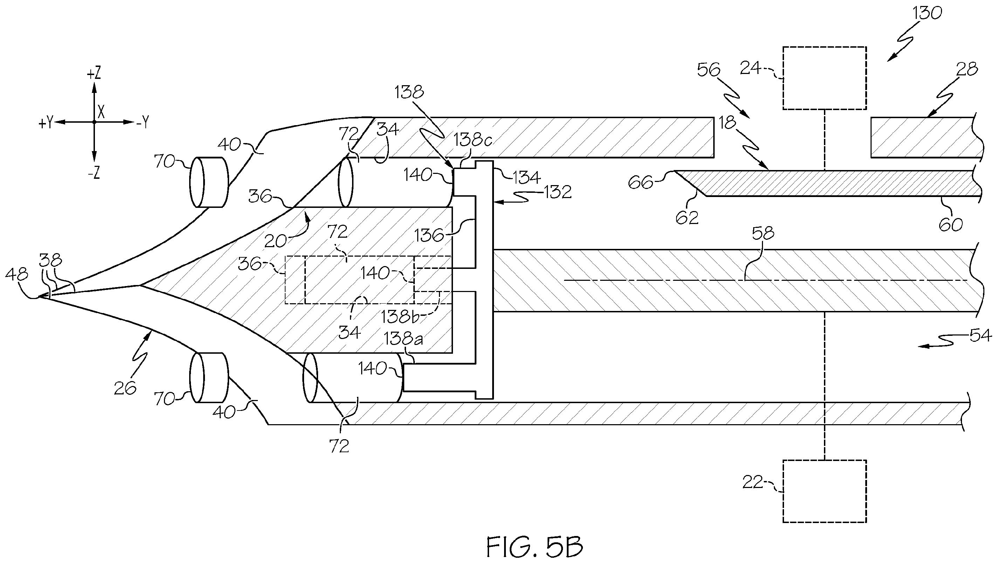

Referring to FIGS. 5A and 5B, another alternative non-claimed biopsy device 130 is depicted. The alternative biopsy device 130 may include the needle 16, the cutting apparatus 18, the marker 20, and the push rod 112 similar to the alternative biopsy device 110 described above, and will not be described again for brevity. The alternative biopsy device 130 may include a deployment device 132 for transferring movement from the push rod 112 to the markers 20. The deployment device 132 may be disposed within the central lumen 54 of the needle body 28. The deployment device 132 may include a first surface 134, an opposite second surface 136, and a plurality of prongs 138 extending from the second surface 136. The plurality of prongs 138 may extend from the deployment device 132 along the center axis 58 toward the plurality of openings 36 in the needle tip 26. The plurality of prongs 138 may be spaced apart and disposed on the second surface 136 such that each prong is aligned with one of the plurality of openings 36. Each of the plurality of prongs 138 may include a contact surface 140 spaced apart from the second surface 136 that is configured to contact one of the push pellets 72. The contact surface 140 of each prong is configured to contact one of the push pellets 72 of the plurality of markers 20 when the push rod 112 is in the deployment position. The deployment device 132 may include a number of prongs 138 equal to the number of openings 36 in the needle tip 26.

The plurality of prongs 138 may include a first prong 138a, a second prong 138b, and a third prong 138c. The first prong 138a may have a first length, the second prong 138b may have a second length, and the third prong 138c may have a third length. Each of the first length, the second length, and the third length may be defined by a distance between the contact surface 140 of the respective prong 138 and the second surface 136 of the deployment device 132. The first length may be greater than the second length, and the second length may be greater than the third length.

When the push rod 112 moves from the retracted position toward the deployment position, the distal end 120 of the push rod 112 may contact the first surface 134 of the deployment device 132 to move the deployment device 132 in the longitudinal direction (e.g., in the +Y direction) toward the openings 36 with the movement of the push rod 112. The first prong 138a may contact the respective push pellet 72 to move the push pellet 72 along with the marker pellet 70 in the longitudinal direction (e.g., in the +Y direction) through the opening 36 to deploy the marker pellet 70. The deployment device 132 may be further moved by the push rod 112 such that the prong 138b contacts the respective push pellet 72 to move the push pellet 72 along with the marker pellet 70 in the longitudinal direction (e.g., in the +Y direction) toward the opening 36 and deploy the respective marker pellet 70. The push rod 112 may further move the deployment device 132 such that the prong 138c may contact the respective push pellet 72 to move the push pellet 72 along with the marker pellet 70 in the longitudinal direction (e.g., in the +Y direction) toward the opening 36 and deploy the respective marker pellet 70.

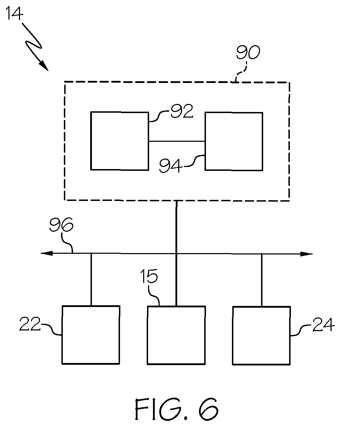

Referring now to FIG. 6, the control system 14 may be operated in conjunction with a biopsy device, such as any of the biopsy devices 10, 110, 130 discussed above. For example, the control system 14 may actuate the first actuator 22 to move the needle tip 26 of the biopsy device 10, or the push rod 112 of the biopsy device 110 or the biopsy device 130. However, for brevity, the control system 14 will only be described with reference to the biopsy device 10.

The control system 14 may include a controller 90 and a communication path 96 communicatively coupling the controller 90 to the first actuator 22, the second actuator 24, and the inputs 15. The controller 90 includes a processor 92 and a non-transitory electronic memory 94 to which various components are communicatively coupled. In some embodiments, the processor 92 and the non-transitory electronic memory 94 and/or the other components are included within a single device. In other embodiments, the processor 92 and the non-transitory electronic memory 94 and/or the other components may be distributed among multiple devices that are communicatively coupled. The controller 90 includes non-transitory electronic memory 94 that stores a set of machine-readable instructions. The processor 92 executes the machine-readable instructions stored in the non-transitory electronic memory 94. The non-transitory electronic memory 94 may include RAM, ROM, flash memories, hard drives, or any device capable of storing machine-readable instructions such that the machine-readable instructions can be accessed by the processor 92. Accordingly, the control system 14 described herein may be implemented in any conventional computer programming language, as preprogrammed hardware elements, or as a combination of hardware and software components. The non-transitory electronic memory 94 may be implemented as one memory module or a plurality of memory modules.

The processor 92 may be any device capable of executing machine-readable instructions. For example, the processor 92 may be an integrated circuit, a microchip, a computer, or any other computing device. The non-transitory electronic memory 94 and the processor 92 are coupled to the communication path 96 that provides signal interconnectivity between various components and/or modules of the actuation system. Accordingly, the communication path 96 may communicatively couple any number of processors with one another, and allow the modules coupled to the communication path 96 to operate in a distributed computing environment. Specifically, each of the modules may operate as a node that may send and/or receive data. As used herein, the term "communicatively coupled" means that coupled components are capable of exchanging data signals with one another such as, for example, electrical signals via conductive medium, electromagnetic signals via air, optical signals via optical waveguides, and the like.

As schematically depicted in FIG. 6, the communication path 96 communicatively couples the processor 92 and the non-transitory electronic memory 94 of the controller 90 with a plurality of other components of the control system 14. For example, the control system 14 depicted in FIG. 6 includes the processor 92 and the non-transitory electronic memory 94 communicatively coupled with the first actuator 22, the second actuator 24, and the inputs 15. The controller 90 may be configured to actuate each of the first actuator 22 and the second actuator 24. The controller 90 may be configured to receive a signal from the inputs 15 indicative of a desire to actuate the first actuator 22, the second actuator 24, or both. The inputs 15 may be any interface capable of receiving an input from a user and sending a corresponding signal to the controller 90, such as, for example, buttons, a touchscreen interface, or the like. When the controller 90 receives the signal from the inputs 15 indicative of a desire to actuate the first actuator 22, the second actuator 24, or both, the controller 90 may actuate the respective first actuator 22, the second actuator 24, or both.

In embodiments, the biopsy device 10 may be manually actuated, such as using a physical toggle to control the movement of the cutting apparatus 18 and the needle tip 26. In such embodiments, the biopsy device 10 may not include the control system 14, or specific components of the control system 14 such as the first actuator 22 and/or the second actuator 24.

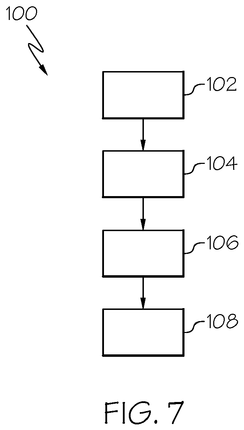

Referring now to FIGS. 3A, 3B, and 7, a flowchart of a non-claimed method 100 of operating a biopsy device, such as any of the biopsy devices 10, 110, 130 discussed above, is depicted. However, the method 100 will only be described with reference to the biopsy device 10 for brevity.

With particular reference to FIG. 3B, the needle tip 26 may initially be in the undeployed position and the cutting apparatus 18 in the closed position. When the cutting apparatus 18 is in the closed position, the cutting apparatus 18 may separate the opening 56 in the needle body 28 from the central lumen 54 in the needle body 28 to prevent unwanted tissue from entering the central lumen 54 during insertion of the biopsy device 10 into the subject's body.

At step 102, the method 100 may include positioning the biopsy device 10 at a target site. The biopsy device 10 may be inserted into the subject's body using the needle tip 26 to puncture tissue and maneuver the needle tip 26 to the target location. When the needle tip 26 is positioned within the subject's body, the resorbable pellet 74 may be resorbed by the subject's body to allow the marker pellet 70 to be moved out of the open lumen 34 through the opening 36 in the needle tip 26.

With reference to FIGS. 3A and 7, at step 104, when positioned at the target site, the method 100 may include actuating the second actuator 24 to move the cutting apparatus 18 in the longitudinal direction (e.g., in the -Y direction) to the open position, thereby allowing tissue at the target site to enter the central lumen 54 through the opening 56 in the needle body 28. Pressure from the surrounding tissue may cause the tissue to enter the central lumen 54 of the needle body 28. However, it is contemplated and possible that the tissue may be drawn into the central lumen 54 in other manners. For example, alternatively or additionally to the pressure from the surrounding tissue, a vacuum source may be connected to the central lumen 54 and operated to draw the tissue into the central lumen 54. The second actuator 24 may then be actuated to move the cutting apparatus 18 in the longitudinal direction (e.g., in the +Y direction) to the closed position. When moving from the open position to the closed position, the edge 66 of the cutting apparatus 18 may puncture and separate the tissue sample positioned within the central lumen 54 from the surrounding tissue. With reference to FIG. 3B, when the cutting apparatus 18 is again positioned in the closed position, the cutting apparatus 18 separates the opening 56 from the central lumen 54 in the needle body 28 to prevent ingress of tissue, fluids, or the like, as well as egress of the tissue sample in the central lumen 54 through the opening 56.

With reference to FIGS. 3B and 7, at step 106, the method 100 may include actuating the first actuator 22 to move the needle tip 26, while the cutting apparatus 18 is positioned in the closed position, to a deployed position, thereby causing the edge 66 of the cutting apparatus 18 to contact the push pellet 72 of the marker 20 to advance the push pellet 72 and the marker pellet 70 through the open lumen 34 toward the opening 36 in the longitudinal direction (e.g., in the +Y direction) to deliver the marker pellet 70 to the target site. The contact between the push pellet 72 and the edge 66 of the cutting apparatus 18 while moving to the deployed position moves the push pellet 72, along with the marker pellet 70, along the open lumen 34 in the longitudinal direction (e.g., in the +Y direction) toward the opening 36 in the needle tip 26. When in the deployed position, the marker pellet 70 is pushed out of the opening 56 via the push pellet 72 and positioned at the target site. The marker pellet 70 may be positioned at the target site to allow a location of where the tissue was removed to be determined.

At step 108, the method 100 may include maneuvering the biopsy device 10 to a second target site to take another tissue sample. The method 100 may repeat the steps 102-106 recited above for taking additional tissue samples.

It should now be understood that embodiments of the present disclosure are directed to devices, systems, and methods for marking a location of a target site. A biopsy device including a needle having a needle body and a needle tip with an opening, a cutting apparatus, and a marker positioned in the opening of the needle tip may be used to take a tissue sample from the target site and mark the location of the target site. The marker may be positioned within a lumen in the needle tip, with the cutting apparatus aligned with the lumen. The needle tip may be moved by an actuator relative to the cutting apparatus. Movement of the needle tip toward the cutting apparatus causes the cutting apparatus to come into contact with the marker, and deploy the marker out of an opening in the needle tip.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A biopsy device (10) for marking a location of a tissue biopsy, the biopsy device (10) comprising:
a needle (16) comprising a needle body (28) and a needle tip (26) movably coupled to the needle body (28), the needle tip (26) defining an open lumen (34);
a cutting apparatus (18) disposed within the needle body (28); and
a marker positioned within the open lumen (34), the marker comprising a marker pellet (70), wherein the needle tip (26) is movable between a undeployed position and a deployed position in which the needle tip (26) is positioned closer to the cutting apparatus (18) than in the undeployed position such that the cutting apparatus (18) interacts with the marker to push the marker pellet (70) through the open lumen (34).

2. The biopsy device (10) according to claim 1, wherein:
the marker further comprises a push pellet (72) positioned proximal to the marker pellet (70) within the open lumen (34), and
in the deployed position, the cutting apparatus (18) contacts the push pellet (72) to advance the push pellet (72) and the marker pellet (70) along the open lumen (34).

3. The biopsy device (10) according to claim 1 or claim 2, wherein the marker comprises a plurality of marker pellets (70) positioned within the open lumen (34).

4. The biopsy device (10) according to any one of the preceding claims, wherein the needle tip (26) defines a plurality of open lumens (34), and the plurality of marker pellets (70) are positioned in the plurality of open lumens (34).

5. The biopsy device (10) according to any one of the preceding claims, wherein the open lumen (34) is open at a position adjacent a distal tip (48) of the needle (16).

6. The biopsy device (10) according to any one of the preceding claims, wherein the marker further comprises a resorbable pellet (74) positioned in the open lumen (34) of the needle (16) distal to the marker pellet (70), and the resorbable pellet (74) seals the open lumen (34) for a predetermined period of time prior to resorption of the resorbable pellet (74).

7. The biopsy device (10) according to any one of the preceding claims, wherein the marker pellet (70) comprises a radiopaque material.

## Patentansprüche

1. Biopsievorrichtung (10) zum Markieren eines Orts einer Gewebebiopsie, wobei die Biopsievorrichtung (10) Folgendes umfasst:
eine Nadel (16), die einen Nadelkörper (28) und eine mit dem Nadelkörper (28) beweglich gekoppelte Nadelspitze (26) umfasst, wobei die Nadelspitze (26) ein offenes Lumen (34) definiert;
eine Schneideinrichtung (18), die innerhalb des Nadelkörpers (28) angeordnet ist;
und
einen Marker, der innerhalb des offenen Lumens (34) positioniert ist, wobei der Marker ein Markerpellet (70) umfasst, wobei die Nadelspitze (26) zwischen einer nicht ausgefahrenen Stellung und einer ausgefahrenen Stellung bewegbar ist, in der die Nadelspitze (26) näher an der Schneideinrichtung (18) positioniert ist als in der nicht ausgefahrenen Stellung, sodass die Schneideinrichtung (18) mit dem Marker zusammenwirkt, um das Markerpellet (70) durch das offene Lumen (34) zu schieben.

2. Biopsievorrichtung (10) nach Anspruch 1, wobei:
der Marker weiter ein Schubpellet (72) umfasst, das proximal zu dem Markerpellet (70) innerhalb des offenen Lumens (34) positioniert ist, und
in der ausgefahrenen Stellung die Schneideinrichtung (18) das Schubpellet (72) kontaktiert, um das Schubpellet (72) und das Markerpellet (70) entlang des offenen Lumens (34) voranzubewegen.

3. Biopsievorrichtung (10) nach Anspruch 1 oder Anspruch 2, wobei der Marker eine Vielzahl von Markerpellets (70) umfasst, die innerhalb des offenen Lumens (34) positioniert sind.

4. Biopsievorrichtung (10) nach einem der vorstehenden Ansprüche, wobei die Nadelspitze (26) eine Vielzahl von offenen Lumina (34) definiert und die Vielzahl von Markerpellets (70) in der Vielzahl von offenen Lumina (34) positioniert sind.

5. Biopsievorrichtung (10) nach einem der vorstehenden Ansprüche, wobei das offene Lumen (34) an einer Position offen ist, die einer distalen Spitze (48) der Nadel (16) benachbart ist.

6. Biopsievorrichtung (10) nach einem der vorstehenden Ansprüche, wobei der Marker weiter ein resorbierbares Pellet (74) umfasst, das in dem offenen Lumen (34) der Nadel (16) distal zu dem Markerpellet (70) positioniert ist, und das resorbierbare Pellet (74) das offene Lumen (34) für eine vorbestimmte Zeitdauer vor der Resorption des resorbierbaren Pellets (74) abdichtet.

7. Biopsievorrichtung (10) nach einem der vorstehenden Ansprüche, wobei das Markerpellet (70) ein röntgendichtes Material umfasst.

## Revendications

1. Dispositif de biopsie (10) pour marquer un emplacement d'une biopsie de tissu, le dispositif de biopsie (10) comprenant :
une aiguille (16) comprenant un corps d'aiguille (28) et une pointe d'aiguille (26) accouplée de façon mobile au corps d'aiguille (28), la pointe d'aiguille (26) définissant une lumière ouverte (34) ;
un appareil de coupe (18) disposé dans le corps d'aiguille (28) ; et
un marqueur positionné dans la lumière ouverte (34), le marqueur comprenant une pastille de marqueur (70), dans lequel la pointe d'aiguille (26) est mobile entre une position non déployée et une position déployée dans laquelle la pointe d'aiguille (26) est positionnée plus près de l'appareil de coupe (18) que dans la position non déployée de sorte que l'appareil de coupe (18) interagisse avec le marqueur pour pousser la pastille de marqueur (70) à travers la lumière ouverte (34).

2. Dispositif de biopsie (10) selon la revendication 1, dans lequel :
le marqueur comprend en outre une pastille de poussée (72) positionnée de façon proximale par rapport à la pastille de marqueur (70) dans la lumière ouverte (34), et
dans la position déployée, l'appareil de coupe (18) entre en contact avec la pastille de poussée (72) pour faire avancer la pastille de poussée (72) et la pastille de marqueur (70) le long de la lumière ouverte (34).

3. Dispositif de biopsie (10) selon la revendication 1 ou la revendication 2, dans lequel le marqueur comprend une pluralité de pastilles de marqueur (70) positionnées dans la lumière ouverte (34).

4. Dispositif de biopsie (10) selon l'une quelconque des revendications précédentes, dans lequel la pointe d'aiguille (26) définit une pluralité de lumières ouvertes (34), et une pluralité de pastilles de marqueur (70) sont positionnées dans la pluralité de lumières ouvertes (34).

5. Dispositif de biopsie (10) selon l'une quelconque des revendications précédentes, dans lequel la lumière ouverte (34) est ouverte au niveau d'une position adjacente à une pointe distale (48) de l'aiguille (16).

6. Dispositif de biopsie (10) selon l'une quelconque des revendications précédentes, dans lequel le marqueur comprend en outre une pastille résorbable (74) positionnée dans la lumière ouverte (34) de l'aiguille (16) en position distale par rapport à la pastille de marqueur (70), et la pastille résorbable (74) scelle la lumière ouverte (34) pendant une durée prédéterminée avant la résorption de la pastille résorbable (74).

7. Dispositif de biopsie (10) selon l'une quelconque des revendications précédentes, dans lequel la pastille de marqueur (70) comprend un matériau radio-opaque.
